# EUROPÄISCHE PATENTSCHRIFT

(11) **EP 0 240 868 B1**
(45) Veröffentlichungstag und Bekanntmachung des Hinweises auf die Patenterteilung: **27.04.1994**
(21) Anmeldenummer: 87104555.5
(22) Anmeldetag: 27.03.1987
(51) Int. Cl.: C07D 211/46, C07H 5/06, C12P 19/26, A61K 31/445

(54) **Verfahren zur Herstellung von 1-Desoxynojirimycin und dessen N-Derivaten**
Process for the preparation of 1-desoxynojirimycine and of its N-derivatives
Procédé de préparation de la 1-désoxynojirimycine et de ses N-dérivés

(30) Priorität: 09.04.1986 DE 3611841
(43) Veröffentlichungstag der Anmeldung: 14.10.1987
(73) Patentinhaber: BAYER AG, 51368 Leverkusen (DE)
(72) Erfinder: Schröder, Theo, Dr., Elkhart, IN 46514 (US); Stubbe, Mathias Dr., D-5600 Wuppertal 11 (DE)

(56) Entgegenhaltungen:
- EP-A- 0 000 947
- EP-A- 0 008 031
- EP-A- 0 012 278
- EP-A- 0 027 908
- EP-A- 0 049 858
- EP-A- 0 055 431
- TETRAHEDRON, vol. 23, 1968, Seiten 2125-2144, Pergamon Press, GB; S. INOUYE et
- al.: "Structure and synthesis of nojirimycin"
- HOUBEN-WEYL, METHODEN DER ORGANISCHEN CHEMIE (1974) BAND XV/1, SEITEN 126, 129, 177
- RöMPP CHEMIE LEXIKON (1989) 9.AUFLAGE, SEITE 236
- CHEMISCHE BERICHTE (1967) 100, 802-819

## Beschreibung

Die Erfindung betrifft ein neues Verfahren zur Herstellung von 1-Desoxynojirimycinen der Formel 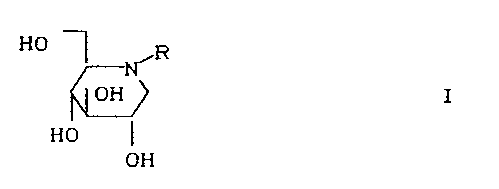
worin
- R: Wasserstoff, C₁-C₁₀-Alkyl, welches durch bis zu 30H- oder C₁-C₄ Alkoxy-Reste substituiert sein kann, bedeutet.

Die Verbindungen der Formel I sind sehr gute α-Glucosidasehemmer und können als Mittel gegen Diabetes verwendet werden, wie aus der Europäischen Patentanmeldung 0 000 947 A 1 bekannt ist.

Aus der DE-OS 2 834 122 ist ein Verfahren zur Herstellung von 1-Desoxynojirimycinen bekannt, bei dem 1-Aminosorbit II mikrobiologisch zur 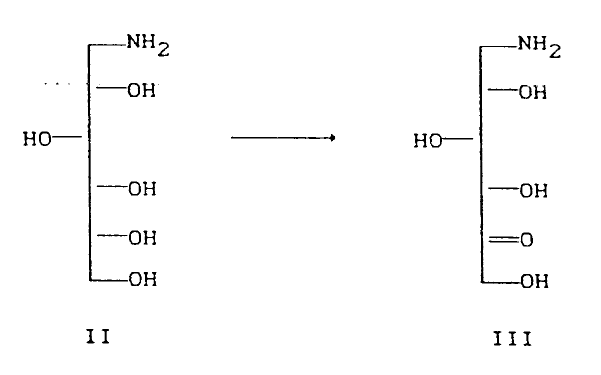
6-Aminosorbose III oxidiert wird, die dann zum 1-Desoxynojirimycin hydriert werden kann. Die Ausbeuten und Volumenausbeuten sind jedoch unbefriedigend. Aus der Europäischen Patentanmeldung 0 049 858 A 2 ist bekannt, daß man Aminosorbosen IV erhält, wenn man Aminosorbite der Formel V mit einer sauer abspaltbaren und bei der mikrobiologischen Oxidation stabilen Gruppe X schützt, die so erhaltenen Verbindungen der Formel VI in an sich bekannter Weise zu geschützten Aminosorbosen VII oxidiert und anschließend die Schutzgruppe mit Säure abspaltet. 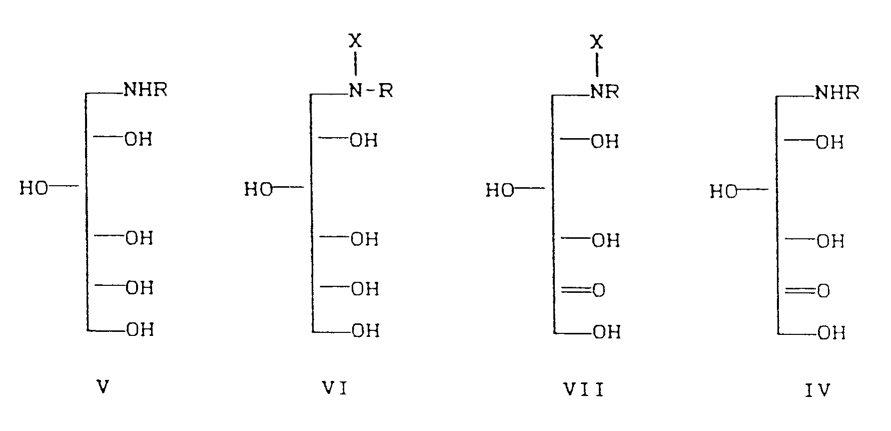
Durch Hydrierung der Verbindungen IV werden Desoxynojirimycine I erhalten.

Weiterhin ist aus der Europäischen Patentanmeldung 0 012 278 A 3 bekannt, daß man Desoxynojirimycine I erhält, wenn man Aminosorbite V mit einer hydrogenolytisch spaltbaren und in der mikrobiologischen Oxidation stabilen Gruppe schützt, die so erhaltenen Aminosorbite VI zu den geschützten Aminosorbosen VII mikrobiologisch oxidiert und anschließend in einem Schritt die Schutzgruppe hydrogenolytisch spaltet und die intermediär entstehenden Aminosorbosen zu Desoxynojirimycinen hydriert. Bei diesem letzten Schritt wird jedoch eine große Menge eines wertvollen Edelmetallkatalysators benötigt.

Alle weiteren bekannten Verfahren zur Herstellung von Desoxynojirimycinen benötigen viele Stufen und aufwendige Reinigungsverfahren. Sie sind in den Europäischen Patentanmeldungen 0 012 278 A 3 und 0 000 947 A 1 zitiert.

Es wurde nun gefunden, daß man Verbindungen der Formel I auf einfachstem Wege und in hohen Ausbeuten erhalten kann, ohne daß eine Reinigung der Aminosorbosen VII oder eine große Katalysatormenge notwendig ist. Dazu wird D-Glucose X in 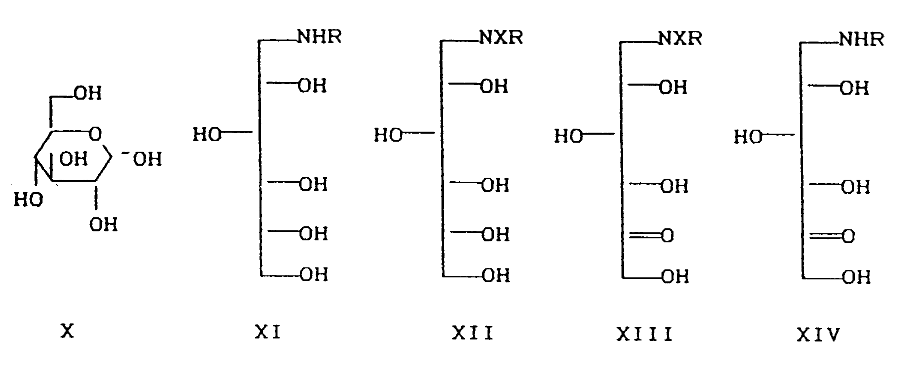
an sich bekannter Weise in die Aminosorbite XI überführt, wobei R die oben angegebene Bedeutung hat und die Aminogruppe mit einer alkalisch abspaltbaren und in der anschließenden mikrobiologischen Oxidation stabilen Gruppe geschützt. Die so erhaltenen Verbindungen XII werden in an sich bekannter Weise mikrobiologisch zu den geschützten Aminosorbosen XIII oxidiert, Anschließend spaltet man die Schutzgruppen alkalisch ab und reduziert die so erhaltenen Aminosorbosen in an sich bekannter Weise katalytisch oder mit komplexen Hydriden zu den Desoxynojirimycinen der Formel I.

Die Erfindung betrifft daher ein Verfahren zur Herstellung von 1-Desoxynojirimycinen der Formel (I) 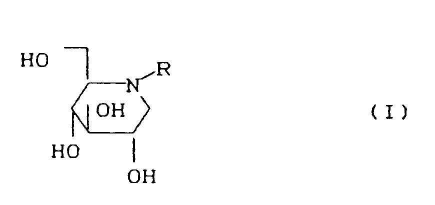
worin
- R: Wasserstoff, C₁-C₁₀-Alkyl, welches durch bis zu 30H- oder C₁-C₄ Alkoxy-Reste substituiert sein kann, bedeutet,
das dadurch gekennzeichnet ist, daß man D-Glucose in an sich bekannter Weise in Aminosorbite (XI) 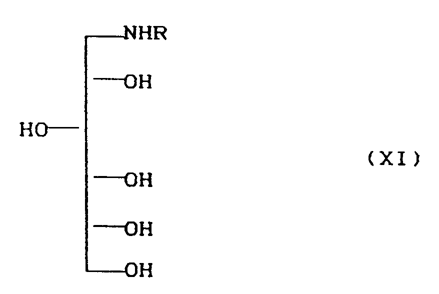
überführt, die Aminogruppe in (XI) mit einer alkalisch abspaltbaren und in der anschließenden mikrobiologischen Oxidation stabilen Gruppe schützt, die geschützten Verbindungen der Formel (XII) 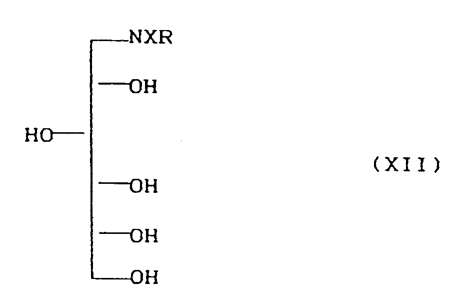
worin X für eine alkalisch abspaltbare Schutzgruppe steht,
mikrobiologisch zu Verbindungen der Formel (XIII) oxidiert 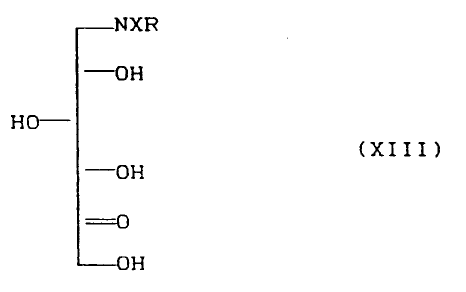
und anschließend die Schutzgruppen alkalisch abspaltet, wobei die Verbindungen der Formel (XIV) erhalten werden, 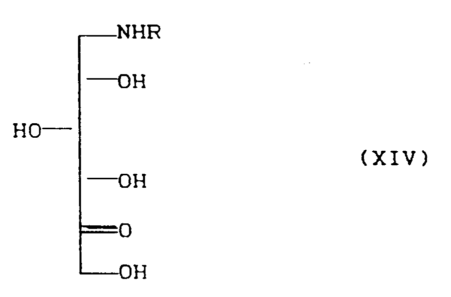
und die so erhaltenen Aminosorbosen in an sich bekannter Weise katalytisch oder mit komplexen Hydriden zu den Desoxynojirimycinen der Formel I reduziert, wobei R die eingangs angegebene Bedeutung hat.

Der Rest R bedeutet vorzugsweise Wasserstoff oder C₁-C₁₀-, insbesondere C₁-C₄-Alkyl. Diese Alkylreste können durch bis zu 3 OH- oder C₁-C₄-Alkoxy-Reste substituiert sein.

Ganz besonders bevorzugt ist die Gruppe der in den Beispielen beschriebenen Verbindungen im Zusammenhang mit deren Herstellungsverfahren.

Ganz besonders bevorzugt bedeutet R Wasserstoff, C₁-C₁₀-Alkyl oder Hydroxyethyl.

Als Schutzgruppen X eignen sich alle Gruppen, die sich alkalisch abspalten lassen und unter den Bedingungen der mikrobiologischen Oxidation beständig sind. Beispielhaft seien folgende Schutzgruppen genannt:
Formyl, Di- und Trihalogenacetyl wie Dichloracetyl, Trichloracetyl.

Besonders bevorzugt sind die Formyl-, die Dichloracetyl- und die Trichloracetylschutzgruppen. Die Herstellung der Zwischenprodukte XII erfolgt im Falle der Amidschutzgruppen in an sich bekannter Weise aus den Aminen XI und den entsprechenden Carbonsäureestern oder Carbonsaureanhydriden in geeigneten Lösemitteln.

Die Verbindungen XII sind besonders für R=H gut kristallisierende Verbindungen, die oft schon während des Ansatzes in der Wärme auskristallisieren und somit in einfachster Weise in hoher Reinheit erhalten werden können.

Für die Herstellung sind sowohl die Carbonsäurealkyl- als auch die -arylester sowie die symmetrischen und gemischten Carbonsäureanhydride geeignet. Als Lösungsmittel sind alle inerten organischen Lösungsmittel und Wasser sowie Mischungen davon geeignet. Beispielhaft seien die Alkohole wie Methanol, Ethanol, Isopropanol und tert. Butanol, Ketone wie Aceton und Methylisobutylketon, Carbonsäureamide wie Dimethylformamid und N-Methylpyrrolidon und Dimethylsulfoxid genannt.

Besonders bevorzugt sind Carbonsäuremethyl- und -ethyl-ester, sowie Wasser, Methanol, Ethanol, Dimethylformamid und N-methylpyrrolidon als Lösungsmittel.

Die Verbindungen der Formel XIII lassen sich sehr einfach in an sich bekannter Weise aus den Verbindungen der Formel XII herstellen, indem man die geschützten Aminosorbite XII in wäßrigen Lösungen mit geeigneten Microorganismen (z.B. Gluconobacter oxidans) oxidiert. Die so erhaltenen Verbindungen sind entweder gut wasserlöslich oder lassen sich durch Extraktion mit geeigneten Lösungsmitteln von den Mikroorganismen abtrennen.

Zur Abspaltung der Schutzgruppen wird die so erhaltene Lösung von XIV gegebenenfalls verdünnt oder aufkonzentriert und mit einer konzentrierten oder verdünnten Base versetzt. Der Verlauf der Abspaltung kann chromatographisch verfolgt werden und ist, abhängig von Art und Menge der verwendeten Base sowie der Schutzgruppe, innerhalb von einigen Minuten bis zu einigen Stunden vollständig. Die Abspaltung kann bei Temperaturen von 0°C bis 60°C erfolgen, bevorzugt ist ein Bereich von 15 bis 30°C.

Als Basen können alle Hydroxide und Carbonate verwendet werden, soweit sie ausreichend in Wasser löslich sind.

Bevorzugt sind die Ammonium-, Alkali- und Erdalkalihydroxide sowie Tetraalkylammoniumhydroxide.

Weiterhin sind basische Ionenaustauscher als Basen verwendbar. Besonders bevorzugt sind stark basische Ionenaustaucher wie z.B. Lewatit® MP 500 OH-Form. Die Austauscherharze können sowohl gelförmig als auch makroporös sein. Der Einsatz kann im batch-Verfahren, d.h. Zugabe des Austauschers zur Lösung von XIV, oder auch in Säulen erfolgen.

Die alkalische Abspaltung der Schutzgruppen führt in praktisch einheitlicher Reaktion zu den Aminosorbosen XIV. Dieses Ergebnis ist nach dem Stand der Technik ausgesprochen überraschend, da nach Paulsen et.al. Chem. Ber. 100, 802 (1967) bekannt ist, daß Aminosorbosen XIV insbesondere mit R=H in stark sauren wäßrigen Lösungen in der Furanose-Form vorliegen und stabil sind, sich jedoch im neutralen Bereich schnell z.B. zum Pyridinderivat XV zersetzen. In alkalischer Lösung liegen sie in der Piperidinose-Form vor und sind nur relativ beständig (siehe Seite 805 unten), d.h. sie sind instabiler als unter sauren Bedingungen . Dieses Zitat enthält nicht den geringsten Hinweis, daß die alkalische Abspaltung eine wesentliche Steigerung der Ausbeute bringt. Weiterhin ist auch überraschend, daß die Aminosorbosen XIV trotzdem in einfacher Weise und in hohen Ausbeuten zu den Verbindungen I reduziert werden können. 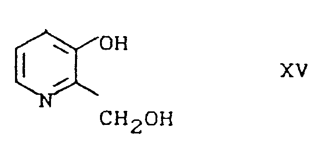
Die Reduktion der Aminosorbosen XIV kann nach an sich bekanntem Verfahren erfolgen. Sie gelingt z.B. durch katalytische Hydrierung an geeigneten Katalysatoren wie in der Europäischen Patentanmeldung 49 858 A 2 beschrieben. Bevorzugt ist jedoch die Reduktion mit komplexen Borhydriden. Besonders bevorzugt sind Natriumboranat, Natriumcyanoborhydrid, Dialkylaminoborane und basische Anionenaustaucher in der BH₄^{⊖}-Form. Ganz besonders bevorzugt sind Natriumboranat NaBH₄, Dimethylaminoboran-BH₄N(CH₃)₂ und die Ionenaustauscher Lewatit M 500, M 600 AP 246, MP 500, MP 600 in der BH₄^{⊖}-Form, Die Verwendung von komplexen Borhydriden ist bekannt und in der Europäischen Patentanmeldung EP 055 431 A 1 beschrieben. Zweckmäßigerweise wird die Reduktion unmittelbar nach beendeter Schutzgruppenabspaltung durchgeführt. Vor Beginn der Reduktion kann der pH-Wert der Lösung auf Werte zwischen pH=1 und pH=14 eingestellt werden. Bevorzugt ist der Bereich zwischen pH 4-13.

Eine besonders einfache Verfahrensweise ergibt sich, wenn man eine mit Alkali stabilisierte Natriumboranat-Lösung zur alkalischen Lösung der Abspaltungsreaktiven gibt. Diese Reduktion im alkalischen Milieu hat weiterhin den Vorteil, daß sich praktisch kein Boranat unter Wasserstoffbildung zersetzt, wie es bei der Reduktion im sauren und neutralen Bereich der Fall ist, und mit Luft gefährliche Knallgasgemische bilden kann,

Dieser besondere Vorteil der alkalischen Spaltung ermöglicht die Reduktion der Aminosorbosen XIII in Rohrreaktoren, die mit basischen Ionenaustauschern in der BH₄^{⊖}-Form gefüllt sind.

Zur Isolierung und Reinigung des Desoxynojirimycins I kann je nach Verfahrensvariante und Substanzeigenschaften entweder direkt aus der gegebenenfalls konzentrierten Ansatzlösung kristallisiert werden oder man zieht die Desoxynojirimycine auf einen sauren Ionenaustauscher auf und eluiert "mit gegebenenfalls verdünnter Säure-, Salz-, Ammoniak- oder Aminlösung" oder man gibt über einen Anionenaustauscher in der OH^{⊖}-Form oder aber man chromatographiert an geeigneten Ionenaustauschern (z.B. Lewatit TSW 40 Na^{⊕}-Form), Kieselgel oder silanisierten Kieselgel, konzentriert jeweils die produkthaltigen Lösungen auf und kristallisiert aus geeigneten Lösungsmitteln um.

Als Ionenaustauscher können prinzipiell alle schwach und stark sauren, sowie schwach und stark basischen Typen eingesetzt werden.

Bevorzugt sind stark saure und stark basische Ionenaustauscher.

Die Durchführung des erfindungsgemäßen Verfahrens ist nicht auf die in den Beispielen genannten Mikroorganismen beschränkt, sondern kann vielmehr auch mit anderen durch den Fachmann in der Natur aufgefundenen oder bei Hinterlegungsstellen erhältlichen oxidierenden Stämmen durchgeführt werden.

### Beispiel 1

Herstellung von N-Formyl-1-amino-1-desoxy-D-glucitol. Eine Suspension von 1-Aminosorbit (1-Amino-1-desoxy-D-glucitol) (200 g) in Methanol (800 ml) und Methylformiat (102 ml) wird 3 Stunden unter Rückfluß gekocht. Nach kurzer Zeit erhält man eine klare Lösung, aus der alsbald die Kristallisation des Produktes einsetzt. Es wird abgekühlt und das Produkt durch Filtration isoliert.
- Ausbeute:: 212 g (92 % der Theorie)
- Fp.:: 139-141°C

### Beispiel 2

Herstellung von N-Dichloracetyl-1-amino-1-desoxy-D-glucitol.
Die Herstellung erfolgt analog Beispiel 1 aus 1-Aminosorbit und Methyldichloracetat.
- Ausbeute:: 86 % der Theorie
- Fp.:: 167-169°C.

### Beispiel 3

Herstellung von N-Trichloracetyl-1-amino-1-desoxy-D-glucitol.
Die Herstellung erfolgt analog Beispiel 1 aus 1-Aminosorbit und Methyldichloracetat.
- Ausbeute:: 83 % der Theorie

| C₈H₁₄Cl₃NO₆ (326,6) | | | | |
|---|---|---|---|---|
| Ber.: | C 29,4 % | H 4,3 % | N 4,3 % | Cl 32,6 % |
| Gef.: | C 29,7 % | H 4,3 % | N 4,4 % | Cl 32,2 %. |

### Beispiel 4

Herstellung von N-Formyl-N-methyl-1-amino-1-desoxy-D-glucitol.
Die Herstellung erfolgt analog Beispiel 1 aus N-Methyl-1-amino-1-desoxy-D-glucitol und Methylformiat.
- Ausbeute:: 85 % der Theorie
- Fp.:: 119-121°C.

### Beispiel 5

Herstellung von N-Formyl-N-hydroxyethyl-1-amino-1-desoxy-D-glucitol.
Die Herstellung erfolgt analog Beispiel 1 aus N-Hydroxy-1-amino-1-desoxy-D-glucitol und Methylformiat. Nach vollständiger Umsetzung wird im Vakuum zur Trockne eingeengt.
- Ausbeute:: 93 % der Theorie Sirup.

### Beispiel 6

Herstellung von N-Dichloracetyl-N-hydroxyethyl-1-amino-1-desoxy-D-glucitol.
Die Herstellung erfolgt analog Beispiel 1 aus N-Hydroxyethyl-1-amino-1-desoxy-D-glucitol und Methyldichloracetat.
- Ausbeute:: 96 % der Theorie Sirup.

### Beispiel 7

Herstellung von N-Formyl-6-amino-6-desoxy-L-sorbose.
Man gibt zu einer Suspension von 40 g Gluconobacter oxydans spp.suboxydans (DSM-50049) in 1 l Leitungswasser, mit Phosphorsäure auf pH4,5 eingestellt, bei 32°C, 700 rpm und einer Belüftung von 3 l Luft/Stunde 200 g N-Formylaminosorbit und nach 4 Stunden weitere 200 g N-Formylaminosorbit. Nach 22 Stunden ist die Umsetzung beendet, die Zellen werden abgeschleudert und verworfen. Das Produkt wird durch Einengen im Vakuum als Öl isoliert.

### Beispiel 8

Herstellung von N-Dichloracetyl-6-amino-6-desoxy-L-sorbose.
Man gibt zu einer Suspension von 40 g Gluconobacter oxydans ssp.suboxydans (DSM 50049) in 1 l Leitungswasser, mit Phosphorsäure auf pH 4,5 eingestellt, bei 32°C, 700 rpm und einer Belüftung von 3 l Luft/Stunde 20 g N-Dichloracetylaminosorbit. Die Reaktion ist nach 10 Stunden beendet, wonach die Zellen abzentrifugiert, 2 mal mit je 100 ml Methanol gewaschen und verworfen werden. Die Methanolextrakte werden mit dem wäßrigen Überstand vereinigt und im Vakuum zur Trockne eingeengt.

### Beispiel 9

Herstellung von N-Trichloracetyl-6-amino-6-desoxy-L-sorbose.
Die Umsetzung und Isolierung erfolgt bei einer Reaktionsdauer von 24 Stunden und einer Substratmenge von 55 g des entsprechenden Sorbits wie in Beispiel 8 beschrieben.

### Beispiel 10

Herstellung von N-Formyl-N-methyl-6-amino-6-desoxy-L-sorbose. Die Umsetzung und Isolierung erfolgt bei einer Reaktions
dauer von 16 Stunden und einer Substratmenge von 10 g des entsprechenden Sorbits wie in Beispiel 8 beschrieben.

### Beispiel 11

Herstellung von N-Formyl-N-hydroxyethyl-6-amino-6-desoxy-L-sorbose.
Die Umsetzung und Isolierung erfolgt bei einer Reaktionsdauer von 45 Stunden und einer Substratmenge von 250 g des entsprechenden Sorbits die in 5 Portionen a 50 g innerhalb von 25 Stunden zugegeben wurden, wie in Beispiel 8 beschrieben,

### Beispiel 12

Herstellung von N-Dichloracetyl-N-hydroxyethyl-6-amino-6-desoxy-L-sorbose.
Die Umsetzung und Isolierung erfolgt bei einer Reaktionsdauer von 28 Stunden und einer Substratmenge von 80 g des entsprechenden Sorbits wie in Beispiel 8 beschrieben.

### Beispiel 13

Herstellung von Desoxynojirimycin aus N-Formyl-6-amino-6-desoxy-L-sorbose.

Zur Lösung von N-Formyl-6-amino-6-desoxy-L-sorbose (aus 0,4 Mol N-Formylaminosorbit) in Wasser (400 ml) wird eine Lösung von Natriumhydroxid (20 g) in Wasser (24 ml) gegeben und zwei Stunden bei 20°C gerührt. Dann wird eine Lösung von Natriumboranat (3,8 g) in Wasser (40 ml), die mit einigen Tropfen 45 %iger Natronlauge stabilisiert worden ist, zugegeben und 1 Stunde bei 20 bis 25°C nachgerührt, Nach Zugabe von Aceton (10 ml) wird die Ansatzlösung über eine Säule mit einem stark sauren Ionenaustauscher (1 l Lewatit SP 112 H^{⊕}-Form) gegeben, mit entsalztem Wasser (zwei Säulenvolumina) nachgewaschen und das Desoxynojirimycin mit 6 %igem Ammoniakwasser wieder eluiert. Die produkthaltigen Lösungen werden im Vakuum zur Trockne eingeengt und das Produkt aus Ethylenglykolmonomethylether kristallisiert.
- Ausbeute:: 33,3 g 51,0 % bezogen auf eingesetzten N-Formylaminosorbit
- Fp.:: 193-194°C.

### Beispiel 14

Herstellung von 1-Desoxynojirimycin aus N-Formyl-6-amino-6-desoxy-L-sorbose.
Zur Lösung der Formylaminosorbose (aus 0,4 Mol N-Formylaminosorbit) in Wasser (400 ml) wird Bariumhydroxyd-Oktahydrat gegeben und 2 Stunden bei 20°C gerührt. Dazu wird nun eine Lösung von Dimethylaminoboran (20 g) in Wasser (200 ml) gegeben und eine weitere Stunde nachgerührt. Der pH-Wert der Ansatzlösung wird mit Schwefelsäure (30 %ig) auf 6,8-7,2 eingestellt und vom ausgefallenen Bariumsulfat abfiltriert.
Das Filtrat wird eingeengt und an Kieselgel chromatographiert (mobile Phase Acetonitril:Wasser:25 %ige Ammoniaklösung 4:1:1 v/v/v).
Die produkthaltigen Lösungen werden im Vakuum zur Trockne eingeengt und der Rückstand aus Wasser/Ethanol umkristallisiert.
- Ausbeute:: 50,2 % bezogen auf eingesetzten N-Formylaminosorbit
- Fp.:: 192-193°C,

### Beispiel 15

Herstellung von Desoxynojirimycin aus N-Formyl-6-amino-6-desoxy-L-sorbose.
Zur Lösung des N-Formylaminosorbose (aus 0,4 Mol Formylaminosorbit) wird in Wasser (400 ml) Lewatit MP 500 OH^{⊖}-Form (1 l) gegeben und eine Stunde bei 20°C gerührt. Dann wird Lewatit MP 500 BH₄^{⊖}-Form (200 ml) zugegeben und eine weitere Stunde bei 20°C gerührt. Der Ionenaustauscher wird abfiltriert und Produktreste werden mit Wasser vollständig ausgewaschen. Das Filtrat wird im Vakuum eingedampft und aus Ethylenglykolmonomethylether kristallisiert.
- Ausbeute:: 53,3 % bezogen auf eingesetzten N-Formylaminosorbit
- Fp.:: 192-193°C.

### Beispiel 16

Herstellung von 1-Desoxynojirimycin aus N-Dichloracetyl-6-amino-6-desoxy-L-sorbose.
Die Herstellung erfolgt analog Beispiel 13 aus N-Dichloracetylaminosorbose.
- Ausbeute:: 53,9 % bezogen auf eingesetzten N-Dichloracetylaminosorbit
- Fp.:: 192-193°C.

### Beispiel 17

Herstellung von Desoxynojirimycin aus N-Dichloracetyl-6-amino-6-desoxy-L-sorbose.
Die Herstellung erfolgt analog Beispiel 14 aus N-Dichloracetylaminosorbose. Als Reduktionsmittel wird Lewatit MP 500 BH₄^{⊖}-Form (200 ml) statt Dimethylaminoboran verwendet.
- Ausbeute:: 53,3 % bezogen auf eingesetzten N-Dichloracetylaminosorbit
- Fp.:: 192-193°C,

### Beispiel 18

Herstellung von Desoxynojirimycin aus N-Trichloracetyl-6-amino-6-desoxy-L-sorbose.
Die Herstellung erfolgt analog Beispiel 13 aus N-Trichloracetylaminosorbose,
- Ausbeute:: 57 % der Theorie, bezogen auf N-Trichloracetylaminosorbit
- Fp.:: 193-194°C.

### Beispiel 20

Herstellung von N-Methyl-1-desoxynojirimycin aus N-Formyl-N-methyl-6-amino-6-desoxy-L-sorbose.
Die Herstellung erfolgt analog Beispiel 13 aus N-Formyl-N-methylaminosorbose.
- Ausbeute:: 46 % der Theorie, bezogen auf eingesetzten N-Formyl-N-methylaminosorbose
- Fp.:: 152-153°C.

### Beispiel 21

Herstellung von N-Hydroxyethyl-1-desoxynojirimycin aus N-Formyl-N-hydroxyethyl-6-amino-6-desoxy-L-sorbose.
Die Herstellung erfolgt analog Beispiel 13 aus N-Formyl-N-hydroxyethylaminosorbose.
- Ausbeute:: 42 % der Theorie, bezogen auf eingesetzten N-Formyl-N-hydroxyethylaminosorbit.

### Beispiel 22

Herstellung von N-Hydroxyethyl-1-desoxynojirimycin aus N-Dichloracetyl-N-hydroxyethyl-6-amino-6-desoxy-L-sorbose.
Die Herstellung erfolgt analog Beispiel 13 aus N-Dichloracetyl-N-hydroxyethylaminosorbose.
- Ausbeute:: 47 % der Theorie, bezogen auf eingesetzten N-Dichloracetyl-N-hydroxyethylaminosorbit.

## Patentansprüche

1. Verfahren zur Herstellung von 1-Desoxynojirimycinen der Formel (I) 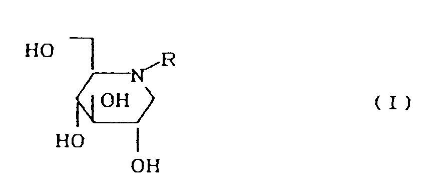 worin
R Wasserstoff, C₁-C₁₀-Alkyl, welches durch bis zu 30H- oder C₁-C₄ Alkoxy-Reste substituiert sein kann, bedeutet,
wobei das Verfahren beinhaltet, daß man D-Glucose in an sich bekannter Weise in Aminosorbite (XI) 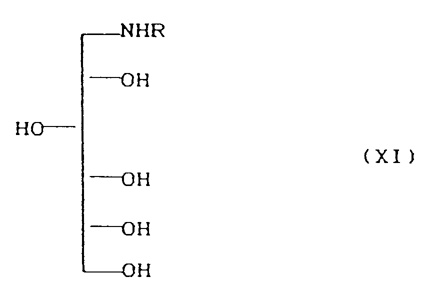 überführt, die Aminogruppe in (XI) mit alkalisch abspaltbaren und unter den Bedingungen der mikrobiologischen Oxidation stabilen Gruppe schützt, die geschützten Verbindungen der Formel (XII) 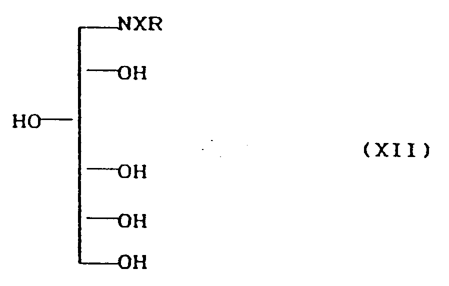 worin X für eine alkalisch abspaltbare Schutzgruppe aus der Gruppe Formyl, Di- und Trihalogenacetyl, steht, mikrobiologisch zu Verbindungen der Formel (XIIII) oxidiert 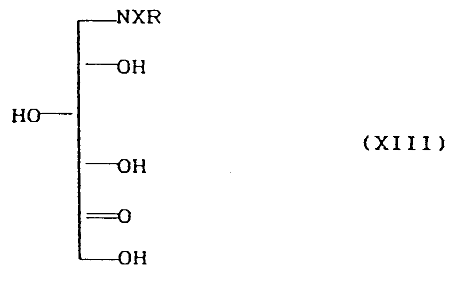 dadurch gekennzeichnet, daß man anschließend die Schutzgruppen alkalisch abspaltet, wobei die Verbindungen der Formel (XIV) erhalten werden, 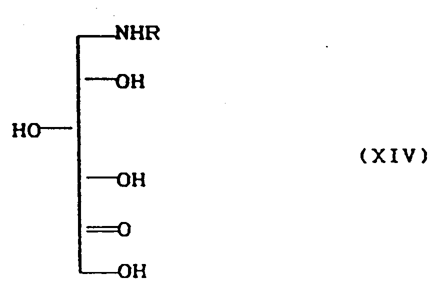 und die so erhaltenen Aminosorbosen in an sich bekannter Weise katalytisch oder mit komplexen Hydriden zu den Desoxynojirimycinen der Formel I reduziert, wobei R die eingangs angegebene Bedeutung hat.

2. Verfahren nach Anspruch 1, dadurch gekennzeichnet, daß man Aminosorbite verwendet, in denen R für Wasserstoff, C₁-C₁₀-Alkyl oder Hydroxyethyl steht.

3. Verfahren nach den Ansprüchen 1 und 2, dadurch gekennzeichnet, daß X in den verwendeten Verbindungen für eine Formyl-, schutzgruppe steht.

4. Verfahren nach den Ansprüchen 1, 2 und 3, dadurch gekennzeichnet, daß man die geschützten Aminosorbite der Formel XII in wäßrigen Lösungen mit Gluconobacter oxidans oxidiert.

5. Verfahren nach den Ansprüchen 1, 2, 3 und 4, dadurch gekennzeichnet, daß man die Schutzgruppe in der Verbindung XIV mit konzentrierter oder verdünnter Base bei Temperaturen von 0°C bis 60°C abspaltet.

## Claims

1. Process for preparing 1-deoxynojirimycins of the formula (I) 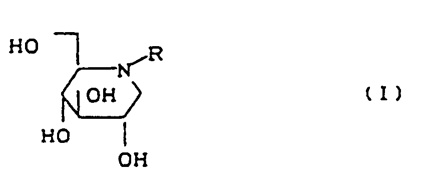 wherein
R denotes hydrogen or C₁-C₁₀-alkyl which may be substituted by up to 3 OH or C₁-C₄-alkoxy radicals,
by converting D-glucose in a manner known per se into aminosorbitols (XI) 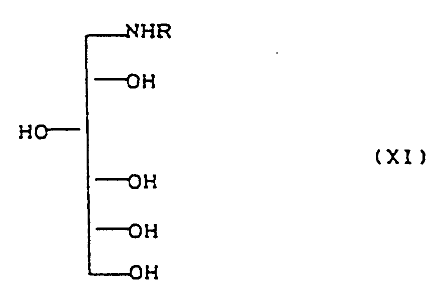 protecting the amino group in (XI) with an alkali detachable group which is stable under the conditions of microbiological oxidation, and microbiologically oxidizing the protected compounds of the formula (XII) 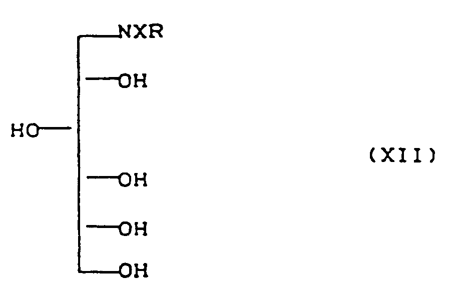 wherein X represents an alkali detachable protective group from formyl, dihaloacetyl and trihaloacetyl to give compounds of the formula (XIII) 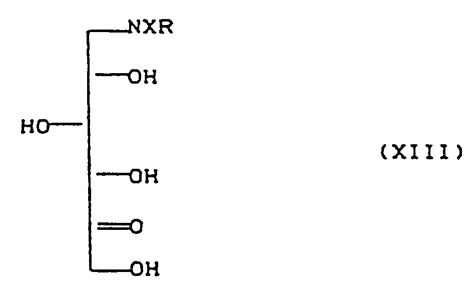 characterized in that the protective groups are subsequently detached with alkali to form the compounds of the formula (XIV) 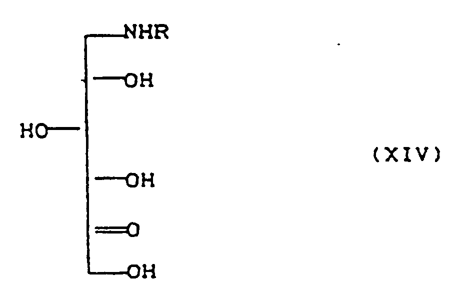 and the aminosorboses thus obtained are reduced in a manner known per se, catalytically or with complex hydrides, to the deoxynojirimycins of the formula I where R is as defined above.

2. Process according to Claim 1, characterized in that, in the aminosorbitols used, R represents hydrogen, C₁-C₁₀-alkyl or hydroxyethyl.

3. Process according to Claims 1 and 2, characterized, in that in the compounds used, the protective group X is formyl.

4. Process according to Claims 1, 2 and 3, characterized in that the protected aminosorbitols of the formula XII are oxidized in aqueous solutions using Gluconobacter oxidans.

5. Process according to Claims 1, 2, 3 and 4, characterized in that the protective group is detached from the compound XIV using concentrated or dilute base at temperatures from 0°C to 60°C.

## Revendications

1. Procédé de préparation des 1-désoxynojirimycines de formule I : 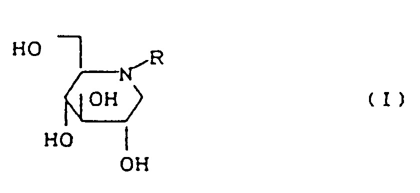 dans laquelle
R représente l'hydrogène ou un groupe alkyle en C₁-C₁₀ qui peut porter jusqu'à trois substituants OH ou alcoxy en C₁-C₄,
dans lequel on convertit le D-glucose de manière connue en soi en aminosorbitols XI 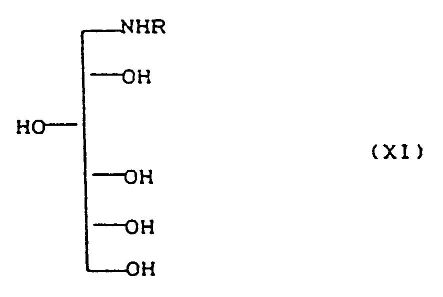 on protège le groupe amino de XI par un groupe stable dans les conditions de l'oxydation microbiologique mais scindable en milieu alcalin, on soumet les composés protégés de formule XII 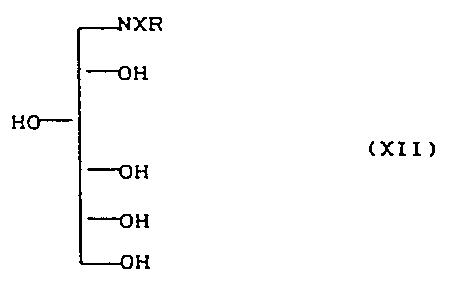 dans laquelle X représente un groupe protecteur scindable en milieu alcalin choisi parmi les groupes formyle, di- et tri-halogénoacétyle,
à oxydation microbiologique donnant les composés de formule XIII 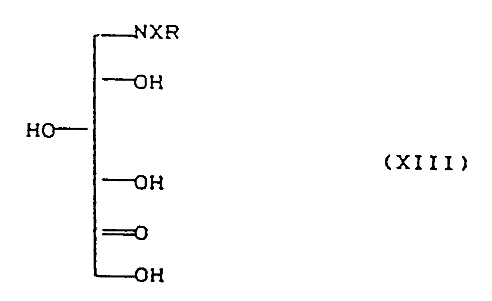 ce procédé se caractérisant en ce que l'on scinde ensuite les groupes protecteurs en milieu alcalin, ce qui donne les composés de formule XIV 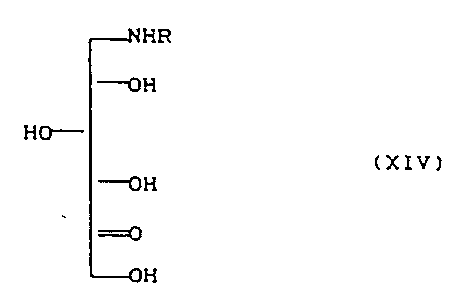 c'est-à-dire des aminosorboses qu'on réduit de manière connue en soi, par hydrogénation catalytique ou à l'aide d'hydrures complexes, en les désoxynojirimycines de formule I dans laquelle R a les significations indiquées ci-dessus.

2. Procédé selon revendication 1, caractérisé en ce que l'on utilise des aminosorbitols pour lesquels R représente l'hydrogène, un groupe alkyle en C₁-C₁₀ ou hydroxyéthyle.

3. Procédé selon les revendications 1 et 2, caractérisé en ce que, dans les composés utilisés, X représente un groupe protecteur formyle.

4. Procédé selon les revendications 1, 2 et 3, caractérisé en ce que les aminosorbitols protégés de formule XII sont oxydés en solution aqueuse par Gluconobacter oxidans.

5. Procédé selon les revendications 1, 2, 3 et 4, caractérisé en ce que, dans le composé XIV, on élimine le groupe protecteur à l'aide d'une base concentrée ou diluée à des températures de 0 à 60°C.
